# EUROPEAN PATENT SPECIFICATION

(11) **EP 1 226 802 B1**
(45) Date of publication and mention of the grant of the patent: **17.08.2005**
(21) Application number: 02001109.4
(22) Date of filing: 23.01.2002
(51) Int. Cl.: A61F 13/49, A61F 13/15

(54) **Disposable diaper**
Wegwerfwindel
Couche-culotte jetable

(30) Priority: 29.01.2001 JP 2001020849; 13.11.2001 JP 2001347889
(43) Date of publication of application: 31.07.2002
(73) Proprietor: Kao Corporation, Chuo-Ku Tokyo 103 (JP)
(72) Inventor: Toyoshima, Haruko, 1 chome, Chuo-ku, Tokyo 103 (JP)
(74) Representative: VOSSIUS & PARTNER

(56) References cited:
- EP-A- 0 487 921
- EP-A- 1 157 681
- EP-A- 1 184 012
- EP-A- 1 197 196
- WO-A-00/76444

## Description

The present invention relates to a disposable diaper which can easily be put on a wearer with its fastening tapes fixed onto either the back side or the front side of the wearer and, in either case, hardly slides down or has a leak while worn.

Known disposable diapers include flat types having fastening tapes and pants types. Flat type disposable diapers are most commonly used for applicability to newborn babies to adults and lower production cost.

While a flat type disposable diaper is easy to fit when a baby is lying still on its back, fitting is not easy when a baby is active enough to try to escape from a diaper, for example, by crawling about. To facilitate diapering such a lively baby, it might be easier to put a diaper back to front so that the fastening tapes may be fixed on the wearer's back side. However, to use a conventional flat type diaper back to front rather makes diapering more difficult. Besides, it is very likely that the diaper, if successfully put back to front, will slide down or leak while worn.

JP-A-1-168902 discloses a flat type disposable diaper having elastic gathers near the base of fastening tapes. JP-A-6-285113, JP-A-6-63077 and JP-A-4-35663 teach a flat type disposable diaper having a fastening tape on an elastic extensible part which is formed of an elastic extensible material and connected to the main body of the diaper. JP-A-U-4-7819 and JP-A-U-4-5826 show a disposable diaper having extensible fastening tapes. All these disposable diapers are difficult to put back to front on a wearer, particularly a crawling infant.

JP-A-2000-262557 discloses a disposable diaper having fastening tapes on both the stomach side and the back side so that a diaperer can put it on a baby lying either on its back or face. However, this diaper has a risk of sliding down while worn or leaking.

JP-W-9-507409 proposes a disposable diaper having fasteners on a pair of side flaps which are made of an elastomer material and connected to the main body of the diaper. This diaper is also difficult to put on back to front, and, even if successfully worn back to front, tends to slide down or leak while worn.

An object of the present invention is to provide a disposable diaper which can easily be put on a wearer with its fastening tapes fixed onto either the back side or the front side of the wearer and, in either case, hardly slides down or has a leak while worn.

The present invention provides a disposable diaper which has a substantially elongate configuration and comprises a main body having a liquid-permeable topsheet, a liquid-impermeable backsheet, and a liquid-retentive absorbent member interposed between the topsheet and the backsheet and a fastening tape provided on each side edge of the main body, the waist opening portion and the under-waist portion in the portion having the fastening tapes being provided with a waist elastic member and a plurality of under-waist elastic members, respectively, in the width direction of the diaper, and both longer side portions of the diaper being provided with a pair of leg elastic members, wherein the under-waist elastic members are fixedly disposed in at least areas extending outwardly from each longer side of the absorbent member in their stretched state so as to manifest elastic contractibility but are not disposed in at least the middle of the area where the absorbent member exists, and the under-waist portion has a greater extension stress than the waist opening portion in the width direction of the diaper.

The under-waist elastic members are elastic members which are disposed in the under-waist portion of the portion having fastening tapes along the width direction of the diaper. They are fixed in their stretched state so as to develop elastic contractibility in at least areas outside the area having the absorbent member. The end of each under-waist elastic member nearer the middle of the diaper width does not reach the middle of the diaper width.

The present invention will be more particularly described with reference to the accompanying drawings, in which:
Fig. 1 is a perspective view of an embodiment of the disposable diaper according to the present invention;
Fig. 2 is a plan view of the disposable diaper of Fig. 1 in the flat-out stretched state with a part cut away;
Fig. 3 is a drawing used to describe the method of measuring an extension stress of the waist opening portion and under-waist portion in the portion having fastening tapes; and
Fig. 4 is a perspective view of the disposable diaper of Fig: 1 which is being put on a baby with its portion having fastening tapes applied to the stomach side, and the fastening tapes being fixed on the back side.

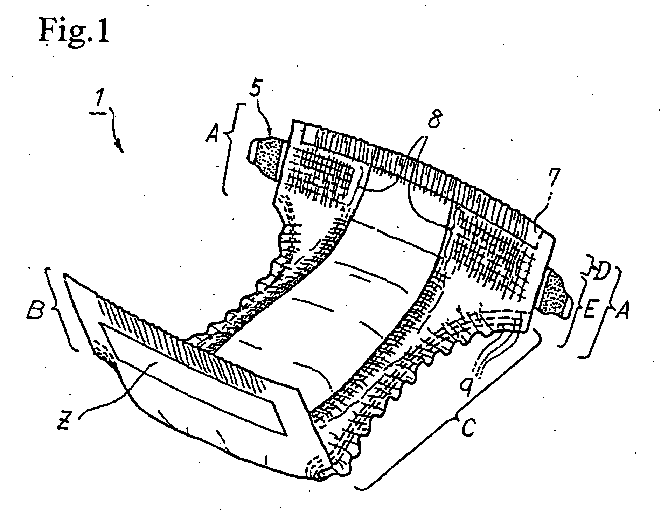

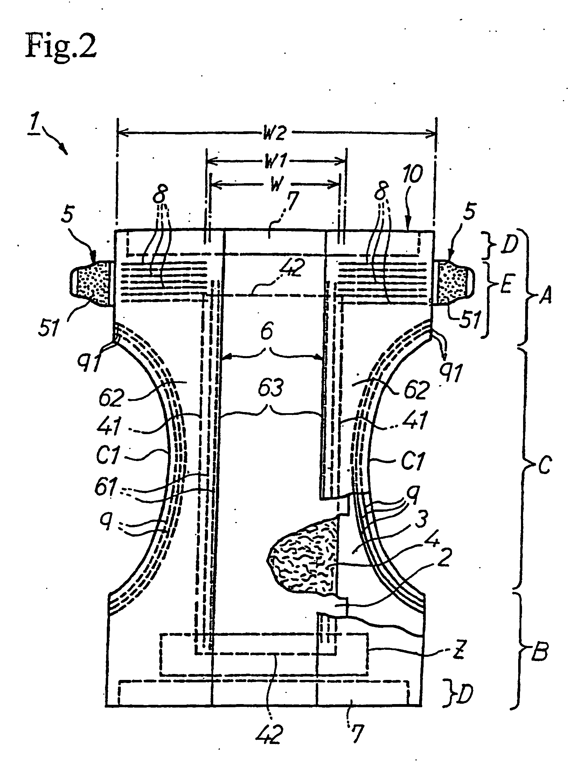

A preferred embodiment of the disposable diaper according to the present invention is illustrated in Figs. 1 and 2. As shown, the disposable diaper 1 is a flat type disposable diaper, which has a substantially elongate configuration and comprises a liquid-permeable topsheet 2, liquid-impermeable backsheet 3, and a liquid-retentive absorbent member 4 disposed between the topsheet 2 and the backsheet 3. The disposable diaper 1 has, in the order from one end to the other in a longitudinal direction of the diaper, a portion A provided with a pair of fastening tapes 5, 5 (this portion will be called "a rear portion A" for the sake of convenience), a crotch portion C, and a portion B (this portion will be called "a front portion B" for the sake of convenience).

In detail, the disposable diaper 1 has both side edges C1 and C1 of the crotch portion C curved inward to make an hourglass-like shape. The topsheet 2 is almost rectangular, larger in size than the absorbent member 4, and is arranged in the middle of the width of the backsheet 3. The backsheet 3 has an hourglass-like shape to make the contour of the diaper 1.

The topsheet 2 and the backsheet 3 both extend outwardly from every side of the absorbent member 4, i.e., both longer sides 41, 41 and both shorter sides 42, 42, and joined together in each extension. The backsheet 3 further extends outwardly from each longer side of the topsheet 2.

A pair of upstanding cuffs (or three dimensional gather) 6 are provided on longer sides of the disposable diaper 1. Each cuff 6 is formed by a sheet 62 having elastic members 61 which is disposed on the area outside the topsheet 2 with an overlap (in its flat-out state as shown in Fig. 2) on the longer side of the topsheet 2. The part of the sheet 62 farther from the middle of the diaper than the base (not shown) of the upstanding cuff 6 is fixed on the topsheet 2 or the backsheet 3. The base end of the cuff 6 is formed near and along the longer side 41 of the absorbent member 4. The part of the sheet 62 nearer to the middle of the diaper than the base of the cuff 6 is fixed on the backsheet 3 only at both ends (shorter sides) of the diaper 1 so that this part has a free end 63. A plurality of elastic members 61 are fixed in their stretched state in this part in parallel to the free end 63, whereby the cuff 6 gathers and stands upright.

The rear portion A having the fastening tapes 5 is divided into a waist opening portion D and an under-waist portion E. The waist opening portion D is provided with a waist elastic member 7 along the diaper width direction, and the under-waist portion E is provided with a plurality of under-waist elastic members 8 and 8 along the diaper width direction.

The waist opening portion D is a portion forming the periphery of the waist opening which is formed when the fastening tapes of the rear portion A are fixed to a landing zone Z of the front portion B (the portion adjacent to the crotch portion C opposite to the rear portion A). The waist opening portion D in each of the rear portion A and the front portion B is provided with a waist elastic member 7 for making a waist gather in its stretched state along the width direction of the diaper 1. The waist elastic member 7 used in the diaper 1 shown in Fig. 1 has a belt-like shape having a width of about 5 to 30 mm. It is fixedly held between the backsheet 3 and the topsheet 2 or the sheet 62 in the waist opening portion D.

Taking the plan view of the diaper 1 with all the elastic members stretched, the rear portion A up, and the front portion B down as shown in Fig. 2, the under-waist portion E in the rear portion A is a portion positioned right below the wai st opening portion D and above the crotch portion C (the inwardly curved portion of the opposing longer sides of the diaper which is conformed to the shape around the leg and provided in the crotch portion of the wearer while worn).

In this particular embodiment shown in Fig. 1, a plurality of the under-waist elastic members 8 and 8 are each composed of a plurality of elastic pieces arranged in parallel to each other at prescribed intervals in the under-waist portion D in the width direction of the diaper 1.

The under-waist elastic members 8 and 8 are fixedly disposed in at least areas extending outwardly from each longer side 41 of the absorbent member 4 in their stretched state so as to manifest elastic contractibility but are not disposed in at least the middle of the area between the longer sides 41 and 41.

In this embodiment, the under-waist elastic member 8 is arranged in both side areas of the rear portion A (both sides areas of the diaper 1). The part of the under-waist elastic member 8 in the area outside the absorbent member 4 (the area extending outwardly from the longer side 41 of the absorbent member 4) is fixedly held between two limp fixing sheets in its stretched state, and the two fixing sheets having the elastic member 8 therebetween are fixedly held between the backsheet 3 and the topsheet 2 or the sheet 62.

The inward end of each under-waist elastic member 8 slightly overlaps with the longer side of the absorbent member 4. There is no elastic member 8 disposed in the area from the middle of the absorbent member 4 to the vicinity of the longer side edge 41.

The width W (see Fig. 2) of the area having no under-waist elastic member (the area having no contractibility) is preferably at least a half of the width W1 of the absorbent member 4 and not wider than the width W1 and from 1/10 to 4/5 of the whole width W2 of the rear portion A. This range of the width W is desirable for improving ease of diapering either in an ordinary manner (with the rear portion A applied to the back side of a wearer, and the fastening tapes fixed on the stomach side, hereinafter referred to as a front fastening manner) or a back-to-front manner (with the rear portion A applied to the stomach side of a wearer, and the fastening tapes fixed on the back side, hereinafter referred to as a back fastening manner) and also for preventing the disposable diaper from sliding down or leaking while worn.

A pair of leg elastic members 9 and 9 are disposed along the inwardly curved lines on the longer sides of the disposable diaper 1 to make leg gathers. Each leg elastic member 9 is provided in its stretched state from the lateral end of the lower (according to Fig. 2) part of the rear portion A to the lateral end of the upper (according to Fig. 2) part of the front portion B to make an arch in the crotch portion C. In the embodiment shown in Fig. 2 each leg elastic member 9 is composed of a plurality of elastic pieces arranged in parallel to each other.

In the rear portion A having the fastening tapes, the under-waist portion E has a greater extension stress than the waist opening portion D in the width direction of the diaper 1. In order to facilitate diapering either in a front fastening manner or in a back fastening manner and to prevent sliding down and leakage, it is preferred that the ratio of the extension stress Q of the under-waist portion E in the diaper width direction to the extension stress P of the waist opening portion D in the same direction, Q/P, be in the range of from 2 to 20, particularly 2 to 6. For the same purpose, it is preferred for the extension stress Q to be from 100 to 1000 cN, particularly from 200 to 600 cN and for the extension stress P to be from 50 to 400 cN, particularly from 100 to 200 cN.

The "extension stress of the waist opening portion D in the diaper width direction" means a stress generated in extending the part having the waist elastic member in the width direction of the diaper. The "extension stress of the under-waist portion E in the diaper width direction" means a stress generated in extending the part having the under-waist elastic members (the elastic members provided on both side portions of the under-waist portion E) in the width direction of the diaper.

The extension stresses of the waist opening portion D and the under-waist portion E in the diaper width direction are measured according to the following methods.

### 1) Method 1:

Measurement is made with a Tensilon tensile tester Model RTA-100, supplied by Orientec Corp. The initial chuck distance of the tester was set at a length 50 mm shorter than the length of the rear portion A in the diaper width direction in its non-stretched state.

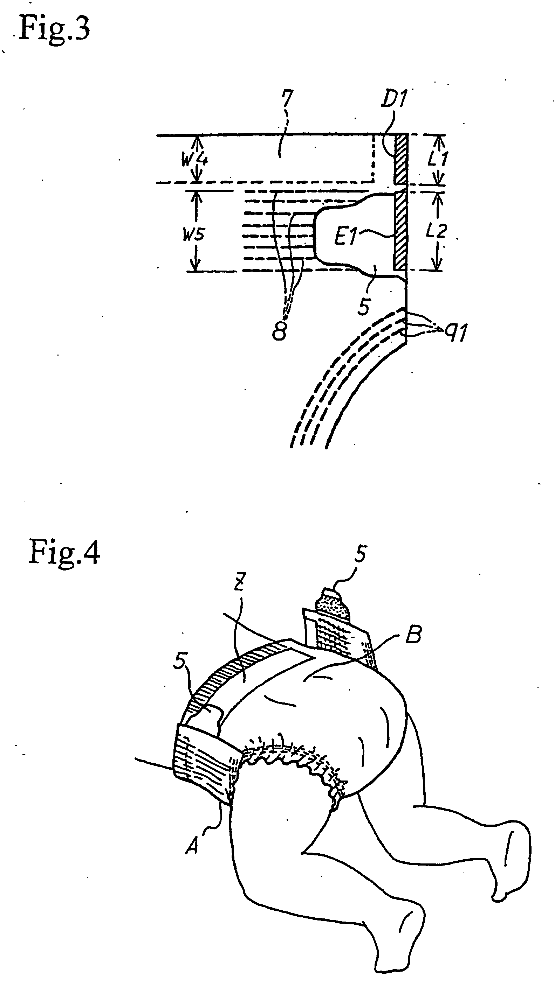

Both lateral ends D1 (see Fig. 3) of the waist opening portion D were fixed to the chucks over a length L1 of 2.5 cm along the longitudinal direction of the diaper. Where the length L1 and the width W4 of the waist elastic member 7 are different, the center of L1 is made to agree with the center of the width W4. Where the waist elastic member 7 is composed of a plurality of pieces, the width W4 is a distance from the upper side of the uppermost piece to the lower side of the lowermost piece. The sample is pulled in the diaper width direction by 90 mm or longer at a cross-head speed of 300 mm/min. The load before extension is subtracted from the load at 90 mm extension to give an extension stress of the waist opening portion D in the diaper width direction.

The extension stress of the under-waist portion E is measured in the same manner as for the waist opening portion D, except that both lateral ends E1 (see Fig. 3) of the under-waist portion E were fixed to the chucks over a length L2 of 5 cm along the longitudinal direction of the diaper.

### 2) Method 2:

Alternatively, the length L1 is made to agree with the width W4, and the length L2 is made to agree with the width W5 of the under-waist elastic member 8, i.e., the length from the upper side of the uppermost elastic piece to the lower side of the lowermost elastic piece.

Having the above-described structure, the disposable diaper according to the present invention can be put on a wearer either in a front fastening manner or a back fastening manner. In either case, hardly does the disposable diaper slide down while worn. Therefore, a diaperer can easily put the disposable diaper on a wearer whatever posture the wearer is in, for example, a lying posture with face up or down or a standing posture with face or back to a diaperer. The diaper can easily be put even on a crawling infant in a back fastening manner as illustrated in Fig. 4.

Since the under-waist elastic member 8 is fixed in its stretched state along the diaper width direction so as to manifest contractibility in the area outside the absorbent member 4 but not in the middle part of the absorbent member 4, the absorbent member 4 is prevented from bunching and gapping. As a result, urine's running on the surface of the diaper can be prevented, and the absorbent member 4 maintains its effective width while worn to exhibit the absorbing performance to the fullest. This advantage makes it feasible to reduce the amount of the absorbent material and the material cost and to make the disposable diaper compact while retaining the same level of absorbing performance. The absorbent member being prevented from bunching, the disposable diaper looks neat while worn either in a front fastening manner or a back fastening manner.

The under-waist elastic member 8 is integrally fixed between sheets making up the main body 10 comprising the topsheet 2, the backsheet 3, and the absorbent member 4. In detail, it is fixed between the backsheet 3 and the sheet 62 forming the upright cuff 6 or the topsheet 2, which constitute the main body 10. Thus, the main body 10 itself is made elastically extensive and contractible. Where an independent elastic member is connected to the main body thereof as disclosed, e.g., in JP-W-9-507409 and JP-A-6-63077, the elastic member tends to be broken when the fastening tape is pulled strongly for fitting the diaper to, for example, a crawling infant in a hurry. The disposable diaper of the present invention has no such a risk because of the above-described integral structure.

Since the pair of leg elastic members 9 and 9 are disposed in inward curves, the disposable diaper 1 is prevented effectively from sliding down while in use. Since the leg gathers fit the legs easily, the disposable diaper 1 can easily be put on a wearer whether the wearer is standing or lying. In particular, because the under-waist elastic member 8 is disposed in an area nearer to the middle in the width direction of the diaper than the end 91 of the leg elastic member 9 positioned in the rear portion A as shown in Fig. 2, the leg opening portion is extended outward together with the outward extension of the under-waist portion E in fitting the diaper 1. As a result, the leg opening portion can be fitted in place easily and in a wearer-friendly manner even when an infant is kicking and struggling to get free. If the under-waist elastic member is disposed only in the area farther than the end of the leg elastic member from the middle of the diaper width as in the disposable diapers disclosed in JP-W-9-507409 and JP-A-6-63077, the leg opening portion having the leg elastic member is not extended in fitting the diaper As a result, the leg opening portions do not fit in place, rather involving a risk of excessively pressing the thighs.

Materials forming the members making up the disposable diaper 1 are then described. The topsheet 2, the backsheet 3, the absorbent member 4, the sheet 62 and the elastic members 61 for making gathered cuffs 6, and the landing zone Z (e.g., a landing tape) can be of any known materials commonly employed in conventional disposable diapers.

The fastening tapes 5 include tapes having a fastening part 51 made of a male member of a mechanical fastener and tapes having a fastening part 51 made of an adhesive. A mechanical fastener is preferred to an adhesive fastener for the following reason. Where a diaperer is in a hurry to put a diaper on, for example, a struggling baby in a back fastening manner, the adhesive part may stick to the diaperer's hand, making fitting difficult. Where a male member of a mechanical fastener is used, the exterior side of the backsheet 3 can be made of a material capable of catching the male member, such as nonwoven fabric, so as to serve as a landing zone Z.

The waist elastic members 7, the under-waist elastic members 8, and the leg elastic members 9 can be of any known elastic materials. The form of these elastic members includes a string, a strip, and a film. Materials making the elastic members include natural rubber, synthetic rubbers (e.g., styrene-butadiene copolymer, polybutadiene, polyisoprene and neoprene), ethylene-vinyl acetate copolymer, extensible polyolefins, and urethane. The waist elastic members 7 are preferably strips of prescribed width. The under-waist elastic members 8 are preferably strings. The leg elastic members 9 are preferably strips. The under-waist elastic members 8, i.e., the left and the right ones in the rear portion A, preferably have 3 to 12 elastic strings each.

The leg elastic members can be disposed in straight lines on both longer sides of the diaper. Each leg elastic member may be made of a single elastic piece.

In the present invention, while "the portion having fastening tapes" has been called "a rear portion" just for the sake of convenience, it does not mean that the portion having fastening tapes is always applied to the back side of a wearer as previously explained. Where the fastening tapes are present in the portion B, the extension stresses as referred to in the present invention are those measured on the waist opening portion and the under-waist portion in the portion B.

The disposable diapers according to the present invention are applicable to adults as well as babies and infants but is more suitable to babies and infants, particularly those who don't like changing their diapers.

The present invention will now be illustrated in greater detail with reference to Examples. The following Examples are presented as being exemplary of the present invention and should not be considered as limiting.

### EXAMPLE 1

Disposable diapers having the structure shown in Figs. 1 and 2 were prepared. The waist opening portion D and the under-waist portion E of the rear portion A had extension stresses of 150 cN and 300 cN, respectively, in the diaper width direction. The under-waist portion E had nine elastic strings arranged over a width (W5; see Fig. of 50 mm as an under-waist elastic member 8 on the left and right hand sides thereof. The waist opening portion D had a 20 mm wide (W4) urethane strip as a waist elastic member 7.

### EXAMPLE 2

Disposable diapers were prepared in the same manner as in Example 1, except that the leg elastic members were disposed in straight lines on both longer sides of the diaper. The waist opening portion D and the under-waist portion E of the rear portion A had extension stresses of 150 cN and 300 cN, respectively, in the diaper width direction.

### COMPARATIVE EXAMPLE 1

Disposable diapers were prepared in the same manner as in Example 1, except that the under-waist portion E had two elastic strings arranged to make a width (W5) of 70 mm as an under-waist elastic member 8 on the left and right hand sides thereof. The waist opening portion D and the under-waist portion E of the rear portion A had extension stresses of 150 cN and 100 cN, respectively, in the diaper width direction.

### COMPARATIVE EXAMPLE 2

Disposable diapers were prepared in the same manner as in Example 1, except that a thin cubic elastic sheet made of urethane (5 cm by 5 cm) was fixed in its non-stretched state on the left and right hand side of the under-waist portion E in place of the elastic members 8. The waist opening portion D and the under-waist portion E of the rear portion A had extension stresses of 100 cN and 0 cN, respectively, in the diaper width direction. Since the urethane elastic member was fixed in its non-stretched state, the under-waist portion E did not extend by 90 mm in the measurement of the extension stress. The sample was equivalent to a commercially sold one. The leg elastic members were disposed in a slight curve.

### COMPARATIVE EXAMPLE 3

Disposable diapers were prepared in the same manner as in Example 1, except that the under-waist elastic members were not provided. The waist opening portion D and the under-waist portion E of the rear portion A had extension stresses of 150 cN and 0 cN, respectively, in the diaper width direction.

In measuring the extension stresses, method 2 was followed in Comparative Example 1, and method 1 was followed in Examples 1 and 2 and Comparative Examples 2 and 3.

### Evaluation of diaper's not sliding down:

To evaluate the diaper's not sliding down while worn, a movable model of a toddler's hips and thighs was used in its standing posture. The model is designed to take a toddling movement and to have artificial urine pored therein and discharged from the crotch.

The disposable diaper prepared in Examples and Comparative Examples was fitted to the model in a front fastening manner or a back fastening manner. The model was made to take a toddling movement at a rate of 150 steps/min for 5 minutes and stopped. While the model was in a still mode, 40 g of artificial urine was poured into the diaper through a tube at a rate of 5 g/min. Then the model was again set to take the same toddling movement for 5 minutes. The 5 minutes' toddling and pouring 40 g artificial urine were repeated alternately until the model took a walking action of 25 minutes in total and a total amount of 160 g of artificial urine was poured. The slide of the diaper was measured for every 5 minutes' toddling movement. The results obtained are shown in Table 1 below.

**TABLE 1**

| | Fastening Side | Extension Stress (cN) | | Slide (mm) | | | | |
|---|---|---|---|---|---|---|---|---|
| | | | | Cumulative Amount of Artificial Urine Poured (g) | | | | |
| | | Waist | Underwaist | 0 | 40 | 80 | 120 | 160 |
| Example 1 | front | 150 | 300 | 5 | 8 | 10 | 15 | 15 |
| | back | | | 0 | 3 | 5 | 5 | 5 |
| Example 2 | front | 150 | 300 | 5 | 10 | 12 | 15 | 15 |
| | back | | | 5 | 8 | 10 | 10 | 12 |
| Comparative Example 1 | front | 150 | 100 | 14 | 21 | 25 | 32 | 35 |
| | back | | | 10 | 16 | 21 | 25 | 30 |
| Comparative Example 2 | front | 100 | 0 | 9 | 14 | 19 | 24 | 25 |
| | back | | | 5 | 9 | 11 | 14 | 15 |
| Comparative Example 3 | front | 150 | 0 | 24 | 30 | 39 | 44 | 45 |
| | back | | | 10 | 20 | 25 | 30 | 40 |

### Evaluation of leak resistance (measurement of dynamic front leak):

The disposable diaper prepared in Examples and Comparative Examples was fitted to the same body model as used above. The diapers of Examples were fitted in the front fastening manner or the back fastening manner, while the diapers of Comparative Examples were fitted in the front fastening manner (an ordinary manner). The model was made to take a toddling movement in its standing posture at a rate of 150 steps/min for 5 minutes and stopped. While the model was in a still mode, 80 g of artificial urine was poured into the diaper through a tube at a rate of 5 g/min. The model was again set to take the same toddling movement for 5 minutes, and a slide (mm) of the diaper was measured. Then, the model with the slid diaper was laid on its stomach, and 40 g of artificial urine was poured. After pouring, the diaper was checked with the naked eye to see if it leaked. Additional 40 g artificial urine was poured into the diaper which did not have a leak at that time, and pouring of 40 g artificial urine was repeated until a leak occurred.

The cumulative amount of poured artificial urine at the time when a leak was observed was taken as "a dynamic front leak". The results obtained are shown in Table 2 below.

**TABLE 2**

| | Fastening Side | Dynamic Front Leak (g) | Slide (mm) |
|---|---|---|---|
| Example 1 | front | 200 | 10 |
| | back | 200 | 5 |
| Comparative Example 1 | front | 160 | 25 |
| Comparative Example 2 | front | 160 | 19 |
| Comparative Example 3 | front | 120 | 39 |

### Evaluation of ease of fitting:

Twenty-eight test users were given 20 samples each of the diapers prepared in Example 1 and Comparative Example 2. They were asked to use the diapers in different ways, that is, for a standing or lying wearer in a usual manner (front fastening) or in a back-to-front manner (back fastening), and to make an evaluation of the ease of fitting by choosing their answers from (a) the first diaper is better (diaper of Example 1), (b) the second diaper is better (diaper of Comparative Example 2), and (c) no difference. The number of the answers (a) or (b) was divided by the total number of the answers, and the quotient was multiplied by 100 to give a consumer acceptance rating (%) of the diaper of Example or the diaper of Comparative Example, respectively. The results obtained are shown in Table 3.

**TABLE 3**

| | Consumer Acceptance Rating (%) | | | | |
|---|---|---|---|---|---|
| Wearer's Posture | Lying Posture | | Standing Posture | | Overall Judgement |
| Fastening Side | Front | Back | Front | Back | |
| Example 1 | 29 | 58 | 61 | 61 | 75 |
| Comparative Example 2 | 29 | 17 | 6 | 4 | 7 |

It is seen from the results in Tables 1 to 3 that the disposable diaper of the present invention is easy to put on a wearer in any posture and hardly slides down while worn and hardly leaks irrespective of whether it is fastened on the wearer's stomach side or back side.

The disposable diaper according to the present invention is easy to put on a wearer in various postures. It hardly slides down while worn and hardly leaks on whichever side (stomach side or back side) it is fastened.

This application claims the priority of Japanese Patent Applications No. 2001-20849 filed January 29, 2001 and No. 2001-347889 filed November 13, 2001.

## Claims

1. A disposable diaper (1) which has a substantially elongate configuration and comprises a main body having a liquid-permeable topsheet (2), a liquid-impermeable backsheet (3), and a liquid-retentive absorbant member (4) interposed between said topsheet and said backsheet and a pair of fastening tapes provided on each side edge of said main body, the waist opening portion (D) and the under-waist portion (E) in the portion having said fastening tapes being provided with a waist elastic member (7) and a plurality of under-waist elastic members (8), respectively, in the width direction of the diaper, and both longer side portions of said diaper being provided with a pair of leg elastic members (9), wherein said under-waist elastic members (8) are fixedly disposed in at least areas extending outwardly from each longer side of the absorbent member (4) in their stretched state so as to manifest elastic contractibility but are not disposed in at least the middle of the area where said absorbent member (4) exists, and said under-waist portion (E) has a greater extension stress than said waist opening portion (D) in the width direction of the diaper.

2. The disposable diaper according to claim 1, wherein said fastening tapes (5, 5) each have a fastening part (51) which mechanically engages with a landing zone (2).

3. The disposable diaper according to claim 1 or 2, wherein said under-waist elastic members are fixed between sheets making up said main body.

4. The disposable diaper according to claim 3, wherein said pair of leg elastic members are each disposed along an inwardly curved line, said under-waist elastic members are disposed in an area nearer to the middle in the width direction of said diaper than the respective ends of said pair of leg elastic members in said portion having said pair of fastening tapes.

## Patentansprüche

1. Wegwerfwindel (1), die einen im wesentlichen länglichen Aufbau hat und einen Hauptkörper mit einer flüssigkeitsdurchlässigen obersten Schicht (2), einer flüssigkeitsundurchlässigen Rückenschicht (3) und einem flüssigkeitsaufnahmefähigen absorbierenden Element (4), das zwischen die oberste Schicht und die Rückenschicht gebracht ist, und ein Paar Verschlußbänder (5, 5), das auf jedem seitlichen Rand des Hauptkörpers vorgesehen ist, aufweist, wobei der Taillenöffnungsabschnitt (D) und der Untertaillenabschnitt (E) in dem Abschnitt mit den Verschlußbändern in der Breitenrichtung der Windel jeweils mit einem elastischen Taillenelement (7) und mehreren elastischen Untertaillenelementen (8) versehen sind, und beide längeren Seitenteile der Windel mit einem Paar elastischer Beinelemente (9) versehen sind, wobei die elastischen Untertaillenelemente (8) zumindest in Bereichen, die sich von jeder längeren Seite des absorbierenden Elements (4) in ihrem gedehnten Zustand nach außen erstrecken, fest angeordnet sind, um die Fähigkeit zum elastischen Zusammenziehen zu zeigen, aber zumindest in der Mitte des Bereichs, in dem das absorbierende Element (4) vorhanden ist, nicht angeordnet sind, und wobei der Untertaillenabschnitt (E) in der Breitenrichtung der Windel eine größere Zugbelastung als der Taillenöffnungsabschnitt (D) hat.

2. Wegwerfwindel nach Anspruch 1, wobei die Verschlußbänder (5, 5) jeweils einen Verschlußteil (51) haben, der mechanisch in eine Anlegezone (Z) eingreift.

3. Wegwerfwindel nach Anspruch 1 oder 2, wobei die elastischen Untertaillenelemente zwischen Schichten befestigt sind, die den Hauptkörper ausmachen.

4. Wegwerfwindel nach Anspruch 3, wobei das Paar elastischer Beinelemente jeweils entlang einer nach innen gekrümmten Linie angeordnet ist, wobei die elastischen Untertaillenelemente in der Breitenrichtung der Windel in einem Bereich näher zur Mitte angeordnet sind als die jeweiligen Enden des Paars elastischer Beinelemente in dem Teil mit dem Paar von elastischen Verschlußbändern.

## Revendications

1. Couche-culotte jetable (1) dotée d'une configuration sensiblement allongée et comprenant un corps principal doté d'une feuille supérieure perméable au liquide (2), d'une feuille inférieure imperméable au liquide (3) et d'un élément absorbant qui retient le liquide (4) interposé entre ladite feuille supérieure et.ladite feuille inférieure et d'une paire de bandes de fixation (5, 5) prévues sur chaque bord latéral dudit corps principal, la partie d'ouverture (D) de la taille et la partie située sous la taille (E) dans la partie comprenant lesdites bandes de fixation qui sont prévues avec un élément élastique (7) à la taille et une pluralité d'éléments élastiques (8) sous la taille, respectivement, dans la direction de la largeur de la couche-culotte, et les deux parties latérales plus longues de ladite couche-culotte étant prévues avec une paire d'éléments élastiques (9) pour les jambes, dans laquelle lesdits éléments élastiques (8) sous la taille sont disposés de manière fixe dans au moins des zones s'étendant vers l'extérieur à partir de chaque côté plus long de l'élément absorbant (4) dans leur état étiré afin de manifester la contraction élastique, mais ne sont pas disposés dans au moins le centre de la zone, dans laquelle ledit élément absorbant (4) existe, et ladite partie sous la taille (E) a une tension d'extension supérieure à ladite partie d'ouverture (D) de la taille dans la direction de la largeur de la couche-culotte.

2. Couche-culotte jetable selon la revendication 1, dans laquelle lesdites bandes de fixation (5, 5) ont chacune une partie de fixation (51) qui se met en prise de manière mécanique avec une zone de réception (Z).

3. Couche-culotte jetable selon la revendication 1 ou 2, dans laquelle lesdits éléments élastiques sous la taille sont fixés entre des feuilles constituant ledit corps principal.

4. Couche-culotte selon la revendication 3, dans laquelle ladite paire d'éléments élastiques pour les jambes sont chacun disposés le long d'une ligne incurvée vers l'intérieur, lesdits éléments élastiques sous la taille sont disposés dans une zone plus proche du centre dans la direction de largeur de ladite couche-culotte que les extrémités respectives de ladite paire d'éléments élastiques pour les jambes dans ladite partie comprenant ladite paire de bandes de fixation.
